# FASCICULE DE BREVET EUROPEEN

(11) **EP 2 633 017 B1**
(45) Date de publication et mention de la délivrance du brevet: **23.11.2016**
(21) Numéro de dépôt: 11832116.5
(22) Date de dépôt: 13.10.2011
(51) Int. Cl.: C11B 9/02, A23L 33/105

(54) **PROCÉDÉ DE FABRICATION D'ARTICLES D'ORIGINE VÉGÉTALE IMPRÉGNÉS DE SUBSTANCE LIQUIDE VÉGÉTALE**
VERFAHREN ZUR HERSTELLUNG VON MIT EINER FLÜSSIGEN MITTEL IMPRÄGNIERTEN ARTIKELN AUS PFLANZISCHER HERKUNFT
METHOD FOR PRODUCING ARTICLES OF PLANT ORIGIN IMPREGNATED WITH A LIQUID PLANT SUBSTANCE

(30) Priorité: 29.10.2010 FR 1058969
(43) Date de publication de la demande: 04.09.2013
(73) Titulaire: Schweitzer-Mauduit International, Inc., Alpharetta, GA 30202-8246 (US)
(72) Inventeur: MOMPON, Bernard, F - 56000 Vannes (FR)
(74) Mandataire: Weber, Martin
(86) Numéro de dépôt international: PCT/FR2011/052393
(87) Numéro de publication internationale: WO 2012/056141

(56) Documents cités:
- WO-A1-94/26853
- GB-A- 2 022 394
- JP-A- 2005 119 967
- JP-A- 2005 306 742
- US-A1- 2010 210 866
- US-B1- 6 818 234

## Description

### Domaine technique

La présente invention se rapporte à un procédé de fabrication d'articles d'origine végétale imprégnés par au moins une substance liquide végétale, ayant la même origine végétale. Avantageusement, le végétal est une plante. L'invention se rapporte également aux articles ainsi fabriqués qui peuvent avoir de multiples applications notamment dans le domaine de l'agroalimentaire, la phytothérapie, la cosmétique, la pharmacie, l'herboristerie, la nutraceutique et la tisanerie.

### Etat de la technique

Les classifications scientifiques classiques regroupent sous le terme végétal plusieurs lignées d'organismes vivants qui, selon l'origine étymologique du terme, végètent.

Les végétaux sont constitués de cellules organisées en feuilles, tiges, racines, fleurs et graines. Les cellules végétales se distinguent par une paroi pectocellulosique faite de cellulose. Toutes les cellules végétales disposent d'une membrane primaire mince et souple qui, lorsque les cellules s'associent, forment une paroi dite secondaire beaucoup plus épaisse et rigide. Les cellules sont alors collées les unes aux autres par une lamelle moyenne riche en pectine. Alors que la paroi primaire est formée de cellulose, hémicellulose, pectine, protéines diverses et de polysaccharides agissant comme liants entre les diverses fibres de cellulose, la paroi secondaire est, elle, beaucoup plus épaisse et plus riche en cellulose que la membrane primaire. Elle contient de la lignine qui est un polymère polyphénolique hydrophobe qui rend la paroi secondaire rigide et très peu perméable à l'eau et aux solvants.

Les végétaux entiers ou parties de végétaux sont sources d'ingrédients divers et de molécules biologiquement actives largement utilisés pour nourrir, soigner, parfumer, améliorer l'hygiène et la beauté des êtres humains. Ces substances d'intérêt peuvent être extraites des végétaux et parties de végétaux par différents procédés physiques.

Cependant, l'extraction industrielle et personnelle (lors de la préparation d'une tasse de thé ou de tisane, par exemple) n'est pas toujours aisée. En effet, elle est généralement incomplète, notamment à cause des parois, écorces, nervures et fibres que possèdent les végétaux mais surtout parce que les parois des cellules végétales, et en particulier la lignine et les fibres de cellulose, s'opposent à l'extraction des substances d'intérêt qui se trouvent dans les cellules végétales, au sein notamment de vacuoles et organites divers, tels que noyaux, mitochondries, chloroplastes, réticulums endoplasmiques, ribosomes et appareil de golgi entre autres.

C'est pourquoi un certain nombre de traitements plus ou moins brutaux doivent généralement être appliqués aux végétaux ou partie de végétaux préalablement à leur extraction afin de rompre cette barrière physique que représentent les parois des cellules végétales. On peut par exemple citer comme procédé permettant de rompre les obstacles mentionnés ci-dessus : le battage, le séchage, la dessiccation, le broyage, la congélation, le cryobroyage, la pelletisation (extrusion), le traitement enzymatique (par exemple par des cellulases, des hemicellulases, des pectinases, des hydrolases), la déstructuration à très haute pression (500 à 5000 bars), la sonication, afin notamment de favoriser l'extraction des substances d'intérêt recherchées.

Toutefois, ces traitements s'avèrent souvent totalement ou partiellement destructifs pour les composants notamment biologiquement actifs contenus dans les cellules végétales.

Il est aussi connu d'adsorber des extraits de certains végétaux sur des supports végétaux, comme décrit dans JP 2005 119967, JP 2005 306742, et GB 2 022 394, par exemple.

Il subsiste donc un réel besoin d'améliorer l'extraction personnelle, c'est-à-dire de disposer facilement et rapidement des substances d'intérêt se trouvant au coeur des cellules des végétaux et ce avec un rendement le plus élevé possible couplé à un minimum de dégradations desdites substances. L'invention vise donc à tendre vers une extraction personnelle la plus complète possible, permettant ainsi d'augmenter la teneur des substances restituées, qui sont à l'origine contenues dans une cellule végétale, par rapport aux solutions existantes dans l'art antérieur.

La présente invention concerne donc un procédé de fabrication d'articles imprégnés d'au moins une substance végétale à partir d'au moins un végétal caractérisé en ce qu'il comprend les étapes suivantes :
a) Extraction et/ou pressage d'au moins un végétal (V1) ou d'au moins une partie dudit végétal produisant un extrait végétal liquide (E1) et un résidu fibreux solide (R1), puis
b) Séparation dudit extrait végétal (E1) dudit résidu fibreux (R1), et
c) Déstructuration dudit résidu fibreux (R1),
d) Fabrication d'une trame fibreuse ou d'un article façonné à partir du résidu fibreux (R1) obtenu à l'étape c),
e) Imprégnation dudit résidu fibreux (R1), avec (i) au moins ledit extrait végétal (E1), éventuellement concentré, purifié, aromatisé et/ou parfumé, avec (ii) au moins une substance végétale hydrosoluble ou liposoluble isolée dudit extrait végétal (E1), avec (iii) au moins une composition comprenant au moins une substance hydrosoluble ou liposoluble dudit extrait végétal (E1) éventuellement concentrée, purifiée, aromatisée et/ou parfumée.

Selon un mode de réalisation dudit procédé, l'étape c) précède l'étape d) qui elle-même précède l'étape e).

Enfin, selon un autre mode de réalisation dudit procédé, l'étape c) précède l'étape e) qui précède l'étape d).

L'invention concerne également un article d'origine végétale susceptible d'être obtenu selon le procédé de l'invention, comprenant une structure solide compacte, homogène, de fibres végétales, ladite structure étant imprégnée par au moins un extrait végétal, éventuellement concentré, purifié, aromatisé, coloré et/ou parfumé, (ii) au moins une substance végétale hydrosoluble ou liposoluble isolée d'un extrait végétal, ou (iii) au moins une composition comprenant au moins une substance hydrosoluble ou liposoluble d'un extrait végétal éventuellement concentrée, purifiée, aromatisée, colorée et/ou parfumée, dans lequel ladite structure et ledit extrait végétal ont la même origine végétale.

### Description de l'invention

Le végétal peut être choisi parmi les plantes alimentaires, les plantes médicinales, les plantes aromatiques et les plantes à parfum.

Parmi les plantes aromatiques, on peut citer le romarin, la sauge, le thym, la menthe, l'origan, le curcuma, le basilic et les clous de girofle.

Parmi les plantes alimentaires, on peut citer le stevia rebaudiana, l'ail, le thé et le café.

Parmi les plantes médicinales, on peut citer le saule, le ginseng, le gingko, la vigne rouge, le thé vert et l'artémisia.

Le végétal peut, par exemple, être choisi parmi les végétaux contenant au moins une substance choisie parmi les antioxydants, les édulcorants, les parfums, les arômes, les caroténoïdes, les xanthophylles, les colorants, les flavonoïdes, les tanins, les polyphénols, les peptides, les vitamines, les protéines, et les principes actifs pharmaceutiques.

Parmi lesdits principes actifs, on peut citer la salicine de l'écorce de saule, les gingkolides des feuilles de gingko biloba, l'hyperforine des somités fleuries du millepertuis, l'artemisinine des feuilles et tiges de artemisia annua, la curcumine des racines de curcuma longa, la genistéïne et la daidzéine des graines de soja, les gingsenoïdes des racines de ginseng, les anthocyanosides et les tanins des feuilles de vigne rouge et les steviosides des feuilles de stevia rebaudiana.

Avantageusement, le végétal est choisi parmi le romarin, la sauge, le thym, la menthe, l'origan, le curcuma, le basilic, les clous de girofle, le Stevia rebaudiana, le thé et le café.

### Brève description des figures

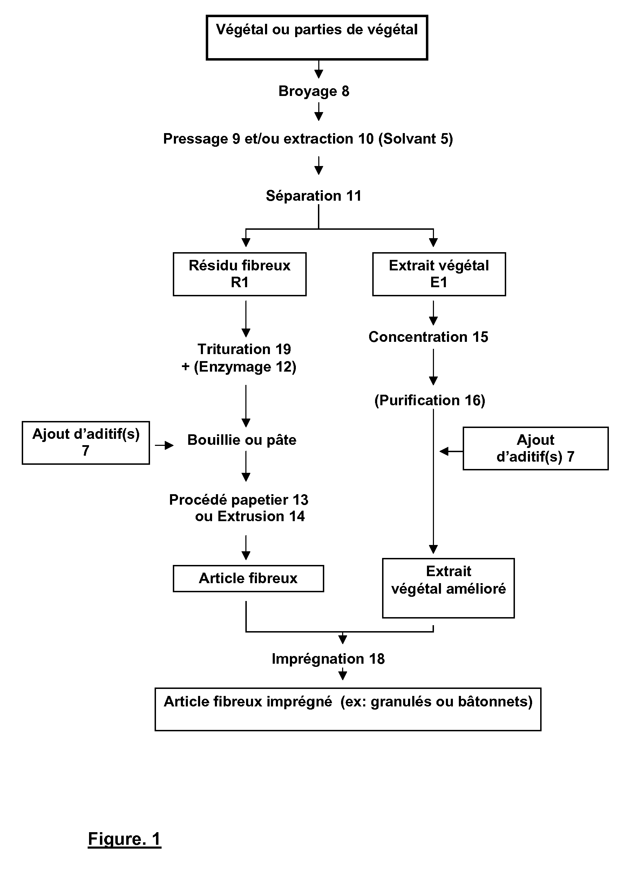

- La **figure 1** représente un procédé selon l'invention dans lequel l'article de résidu fibreux, par exemple un granulé ou un bâtonnet, est imprégné par un extrait végétal postérieurement à sa fabrication.

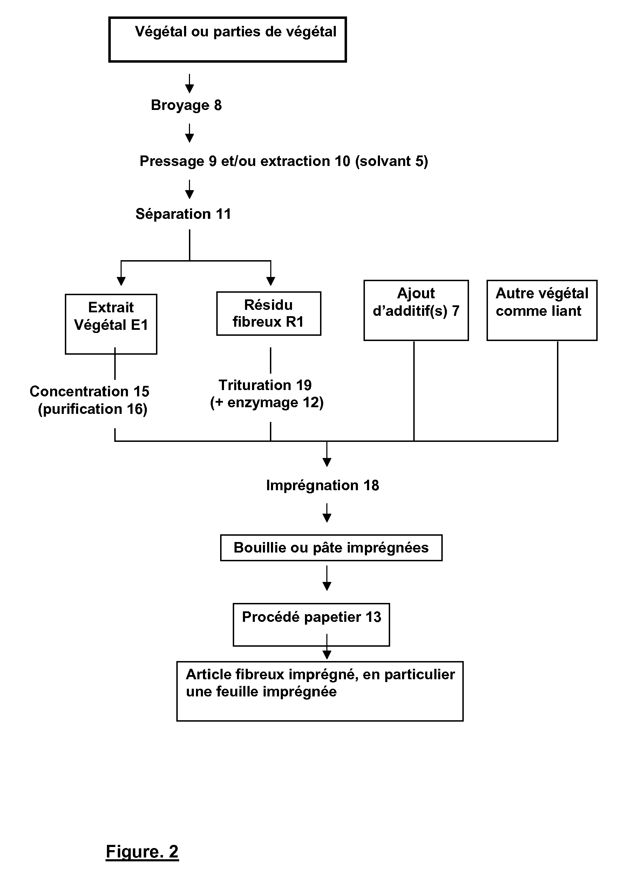

- La **figure 2** représente un procédé selon l'invention dans lequel une bouillie ou une pâte fibreuse fabriquée à partir d'au moins un végétal est imprégnée par au moins un extrait végétal préalablement isolé dudit végétal ou d'un autre végétal (cette deuxième option ne faisant pas partie du cadre de l'invention) et ce préalablement au procédé de mise en forme.

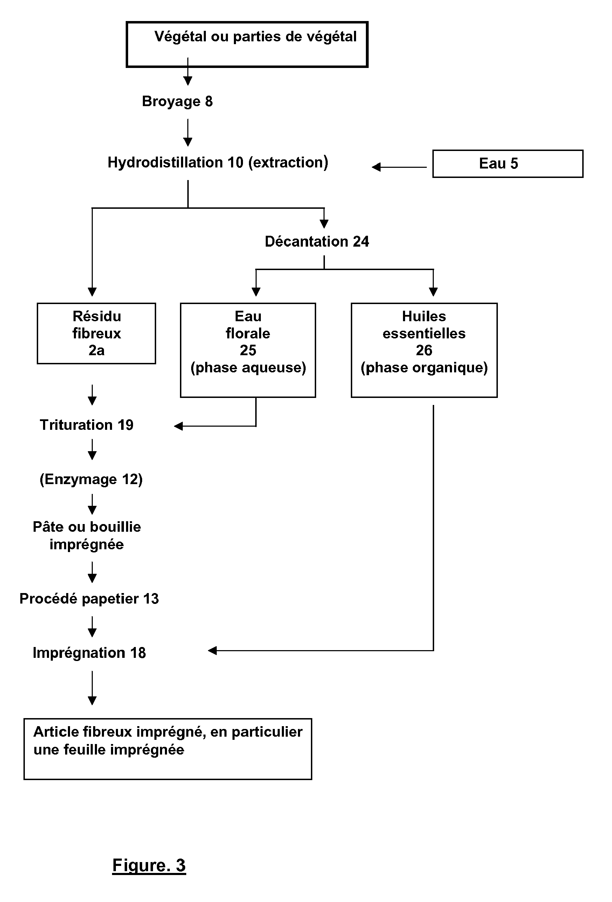

- La **figure 3** représente un procédé selon l'invention dans lequel les huiles essentielles d'un végétal de départ sont extraites par la vapeur d'eau, puis après séparation de la phase aqueuse et de la phase organique, l'eau d'extraction peut être utilisée pour imprégner une bouillie ou une pâte faite à partir du résidu fibreux alors que les huiles essentielles sont éventuellement utilisées pour imprégner les articles constitués par ladite bouillie ou pâte. Lesdits articles peuvent être sous la forme de parties de plantes reconstituées.

Le procédé selon l'invention comprend une étape d'extraction et/ou pressage suivie d'une étape de séparation des produits insolubles ou résidus fibreux (R1) d'un végétal (V1) ou partie d'un végétal des substances solubles ou extrait végétal (E1).

Préalablement aux étapes d'extraction et/ou pressage, une étape optionnelle de broyage/découpage peut être mise en oeuvre. Elle a pour but de déchiqueter le végétal ou partie de végétal et donc de rompre les parois des cellules végétales.

L'extraction et/ou le pressage peut être réalisé(e) sur au moins une partie de végétal, frais, congelé ou sec, choisi parmi les racines, l'écorce, les graines, la tige, les feuilles, les fleurs et les fruits.

L'extraction peut être mise en oeuvre par pressage et/ou par extraction de végétaux, en particulier de plantes, avec au moins un solvant à température ambiante et à pression atmosphérique, ledit solvant pouvant être choisi parmi l'eau, l'éthanol, l'hexane, l'acétone et les hydrofluorocarbones. D'autres procédés d'extraction peuvent être utilisés mettant en jeu au moins un solvant, tel que par exemple le R134a ou alors le dioxyde de carbone et ce à différentes températures, à différentes pressions et différents états (liquides ou gazeux). On peut, par exemple, utiliser un solvant tel que par exemple choisi parmi les solvants mentionnés ci-dessus ou, le cas échéant le dioxyde de carbone :
- à l'état liquide (solvants volatils et non volatils à température ambiante),
- à l'état subcritique (l'eau à une température supérieure à 100°C et une pression supérieure à 1 bar), ou
- à l'état supercritique (le CO₂ à une température supérieure à 31 °C et une pression supérieure à 73 bars).

Le rapport pondéral solvant/végétal est en général de 1 à 10.

Ladite étape de séparation peut être mise en oeuvre par séparation de la phase liquide de la phase solide résultantes par filtration, avec ou sans pression, par centrifugation ou par toutes autres méthodes couramment utilisées au laboratoire.

Selon un mode de réalisation, l'extraction solide-liquide est effectuée en utilisant un solvant dans lequel est mis en contact le végétal ou au moins une partie du végétal, éventuellement préalablement découpé(e) et/ou broyé(e), puis en séparant la phase liquide obtenue de la phase solide constituée par le végétal par filtration avec ou sans pression ou par centrifugation lors d'une étape de séparation.

Selon un autre mode de réalisation, l'extraction solide-liquide est effectuée en hydrodistillant le végétal ou au moins une partie du végétal, éventuellement découpé(e) et/ou broyé(e), avec l'eau comme solvant puis à séparer les phases organique et aqueuse résultantes.

Ces procédés d'extraction peuvent être couplés à ou se présenter sous la forme d'une extraction mettant en oeuvre des micro-ondes tel que par exemple le procédé « Vacuum Microwave Hydrodistillation » abrégé VMHD, le procédé « Microwave Assisted Process » abrégé MAP et le procédé d'extraction par Solvant avec Micro-ondes abrégé ESAM, ou par l'utilisation des ultrasons tel que la sonication.

Ces procédés d'extraction peuvent être continus ou discontinus.

Les procédés et le cas échéant le ou les solvants peuvent être choisis pour satisfaire aux exigences de la réglementation des produits « bio » et être respectueux de l'environnement.

Une fois les substances solubles séparées des résidus fibreux, le procédé selon l'invention peut éventuellement comprendre une étape de concentration des substances solubles ou extrait végétal en une liqueur, et/ou une étape d'isolement d'au moins une substance soluble de l'extrait ou de ladite liqueur, voire une étape de purification ou d'élimination d'éventuelles impuretés présentes de manière à obtenir un extrait purifié, une liqueur purifiée ou au moins une substance soluble, isolée, purifiée dans lesquelles peuvent en outre être incorporé au moins un additif. L'extrait végétal brut d'origine est ainsi transformé en un extrait végétal amélioré, que celui-ci soit sous forme d'extraits sec, d'extrait liquide, de liqueur ou de substance isolée.

Ledit additif peut être choisi parmi (i) les arômes, comme par exemple le menthol, la réglisse, les extraits de fruits en général, (ii) les parfums et (iii) les colorants, comme par exemple le caramel, le rouge de betterave, les anthocyanes et le Cu-chlorophylline.

Les étapes de concentration, d'isolement et de purification se font selon les techniques habituellement utilisées en laboratoire.

Le procédé selon l'invention comprend, en outre, une étape de déstructuration des produits insolubles ou résidus fibreux solides (R1) par un procédé qui peut être mécanique, chimique et/ou biologique, avantageusement un procédé mécanique couplé à un procédé biologique.

Le résidu fibreux peut être traité par un procédé mécanique d'agitation violente ou d'attrition et de délitation mécanique simple, lors d'une étape de trituration pouvant être assistée, par exemple, par des ultrasons. Le résidu fibreux peut ainsi être transformé en une bouillie, c'est-à-dire un appareil liquide inhomogène, avantageusement aqueux, contenant des fibres entières ou partiellement détruites ou en une pâte, c'est-à-dire un appareil liquide homogène, avantageusement aqueux, la consistance de l'appareil dépendant de l'intensité et la durée de cette étape de déstructuration.

Selon un mode de réalisation, l'étape de déstructuration peut avoir lieu par cisaillement mécanique et frottement du résidu fibreux entre un rotor et un stator, éventuellement accompagnés d'ultrasons.

Le résidu fibreux peut être traité biologiquement. Le résidu fibreux peut être (i) ensemencé par des levures, avantageusement des levures choisies dans le genre saccharomyces ou peut être (ii) mélangé à une quantité d'eau suffisante pour former une pâte liquide puis est ensemencé par des enzymes avantageusement des enzymes, choisies parmi les enzymes à activités pectinases, cellulases, amylases et leurs mélanges.

Cette étape biologique a pour but de réduire partiellement la taille des fibres notamment de cellulose, lignine et pectine. Par exemple, une certaine liquéfaction du résidu fibreux et l'élimination de ballast peut être obtenu par l'ajout d'au moins une enzyme choisie par exemple parmi les enzymes définies ci-dessus.

On peut rajouter en outre au résidu fibreux au moins un additif d'origine naturelle, éventuellement choisi parmi (i) les liants tels que par exemple, la gomme arabique et la gomme guar, (ii) les hydrates de carbone d'origine végétale, éventuellement choisis parmi le glucose et les sucres invertis, (iii) les produits de source animale, éventuellement choisis parmi la chitine désacétylée et les gommes de plantes marines, telles que les alginates, les carraghénanes et l'agar-agar, (iv) les fibres de renforcement, comme par exemple des fibres de paille de céréales, de bagasse, de coton, de pin ou d'eucalyptus, (v) les arômes comme par exemple le menthol, la réglisse, les extraits de fruits en général, (vi) les colorants, avantageusement un colorant naturel hydrosoluble choisi par exemple parmi le caramel, le rouge de betterave, les anthocyanes et le Cu-chlorophylline , (vii) les parfums et (viii) les charges diverses.

Le procédé selon l'invention comprend, ensuite, une étape de fabrication d'une trame fibreuse ou d'un article façonné à partir des produits insolubles ou résidu fibreux se présentant sous la forme d'une bouillie ou d'une pâte telle que définie ci-dessus. La fabrication de ladite trame fibreuse peut se faire selon un procédé de type papetier et le façonnage dudit article fibreux peut se faire par extrusion, permettant ainsi d'obtenir un article solide plus ou moins expansé. Lors de l'étape de façonnage les articles façonnés peuvent se présenter sous la forme de feuilles, de bâtonnets, de granulés, de fibres, pleines ou creuses, ou de chips.

Le procédé comprend enfin une étape d'incorporation (également appelé étape d'imprégnation) de (i) ladite liqueur de substances solubles éventuellement purifiées ou (ii) d'au moins ladite substance soluble isolée, éventuellement concentrée ou (iii) d'au moins une liqueur d'origine végétale ou (iv) d'un extrait végétal, à la trame fibreuse ou directement aux résidus fibreux issus de l'étape de séparation, voire à la bouillie ou la pâte, obtenues à l'issu de l'étape de déstructuration, et/ou comprend une étape d'imprégnation des articles façonnés.

Lors de l'étape d'imprégnation e) du résidu fibreux, ce dernière peut être, en outre, imprégnée avec (i) au moins un colorant, avantageusement un colorant naturel hydrosoluble choisi parmi le caramel, le rouge de betterave, les anthocyanes et le Cu-chlorophylline et/ou avec (ii) au moins une fibre alimentaire hydrosoluble d'origine végétale ou animale choisie parmi les carraghénanes, les alginates, les pectines, les amidons et les xanthanes, les caséines et les gélatines, avantageusement une fibre alimentaire hydrosoluble d'origine végétale.

Après séchage, on obtient alors des articles d'origine végétale comprenant une structure solide compacte de fibres végétales, ladite structure étant imprégnée par au moins un extrait végétal, éventuellement concentré, purifié, aromatisé, colorée et/ou parfumé, (ii) au moins une substance végétale hydrosoluble ou liposoluble isolée d'un extrait végétal, ou (iii) au moins une composition comprenant au moins une substance hydrosoluble ou liposoluble d'un extrait végétal éventuellement concentrée, purifiée, aromatisée, colorée et/ou parfumée. L'extrait d'origine végétal peut être choisi parmi les extraits de racines, de graines, de tige, de feuilles, de fleurs, de fruits ou leurs mélanges.

Selon un mode de réalisation de la présente invention relatif à la figure 1, le procédé consiste à séparer d'une part les substances ou ingrédients solubles formant l'extrait végétal (3a) et d'autre part les substances insolubles, c'est à dire les résidus fibreux (2a) d'un végétal en tant que matière première. Pour ce faire, le végétal sous forme sèche, fraîche ou congelée, peut être préalablement broyé et/ou découpé lors d'une étape de broyage/découpage (8).

L'étape de pressage (9) ou d'extraction (10) s'effectue ensuite en utilisant un solvant (5), de préférence de l'eau, un alcool éthylique pur ou en mélange hydroalcoolique ou tout autre solvant adapté pur ou en mélange à froid, à température ambiante ou à une température supérieure au point d'ébullition du ou des solvants utilisés avec éventuellement une macération préalable du végétal, éventuellement broyé préalablement.

Selon une variante du procédé, l'extraction (10) est assistée par les micro-ondes et le vide pulsé (procédé décrit dans le document EP 0 698 076 intitulé « procédé et installation d'extraction sans solvant de produits naturels par les micro-ondes » connu sous le nom de VMHD). Dans ce cas l'extrait végétal obtenu, c'est à dire l'eau de constitution du végétal augmentée des huiles essentielles, est directement réincorporé dans le résidu fibreux. Par ce procédé d'extraction rapide et à faible température, il a été observé l'absence de dégradation des huiles essentielles et des eaux florales obtenues conduisant après imprégnation à une plus grande naturalité notamment olfactive des articles imprégnés selon l'invention.

La séparation (11) des substances solubles formant l'extrait végétal (3a) et les insolubles formant le résidu fibreux est traditionnellement réalisée par filtration au travers d'un média filtrant comme une toile, une grille, ou par centrifugation.

Selon une variante du procédé, le végétal sous forme fraîche ou décongelée et riche en eau (% en poids de l'eau supérieure à 50% par rapport au poids total du végétal) peut être traité directement par pressage (9) et fournir l'extrait végétal (3a) d'une part et les substances insolubles ou résidu fibreux (2a) d'autre part.

L'extrait végétal (3a) est une solution qui peut être concentrée lors d'une étape de concentration (15) par évaporation partielle du solvant sous l'effet combiné du vide et de la température ou par filtration sur une membrane sélective (procédé d'osmose inverse ou d'ultrafiltration) (15).

Si nécessaire, l'extrait végétal (3a) peut être purifié lors d'une étape de purification (16) par passage sur une colonne garnie d'un absorbant ou d'une résine ou purifié par extraction sélective par un solvant non miscible avec l'extrait végétal en solution.

A ce stade, la teneur excessive de certains végétaux en matière minérale (silice, potassium, calcium..) peut en outre être réduite par décantation et précipitation spontanée ou provoquée.

L'Extrait végétal (3a) peut aussi être enrichie par au moins un additif (7) choisi parmi (i) les arômes comme par exemple le menthol, la réglisse, les extraits de fruits en général et (iv) les colorants. On obtient alors un extrait végétal amélioré (3b).

Le résidu fibreux (2a) est traité par un procédé physique d'agitation violente d'attrition et de délitation mécanique simple, lors d'une étape de trituration (19) pouvant être assistée par exemple par des ultrasons et ainsi ledit résidu est transformé en une bouillie aqueuse inhomogène contenant des fibres entières ou partiellement détruites.

Selon une variante du procédé, le résidu fibreux est traité biologiquement lors d'une étape d'enzymage (12) décrite plus haut.

A l'issue de l'étape de trituration (19) ou de l'étape de trituration suivie de l'étape d'enzymage (12), on obtient une pâte définissant un appareil homogène ou une bouillie définissant un appareil inhomogène dû à une trituration grossière et incomplète.

La bouillie ou la pâte peut être alors enrichie par au moins un additif (7) choisi parmi (i) les fibres de renforcement, comme par exemple des fibres de paille de céréales, de coton, ou d'eucalyptus, (ii) les humectants, comme par exemple le glucose et les sucres invertis, (iii) les arômes comme par exemple le menthol, la réglisse, les extraits de fruits en général, (iv) les colorants et (v) les charges diverses.

Puis la bouillie ou la pâte de résidu fibreux peut être transformée par des techniques courantes en papeterie selon un procédé papetier (13), de façon continue ou discontinue, en un article (2b) par exemple une trame fibreuse appelée également trame papetière ou extrudée lors d'une étape d'extrusion (14).

Notamment par un procédé simple, la pâte ou la bouillie est étalée sur une bande métallique sans fin et séchée à l'air formant ainsi une feuille de papier. Mais de nombreux autres procédés de fabrication de feuilles de papier sont connus, par exemple les procédés par stratification ou par pressage.

L'extrait végétal amélioré (3b), éventuellement concentré, purifié, aromatisé et/ou coloré est introduit dans les résidus fibreux par pulvérisation ou tout autre procédé d'imprégnation (18).

Selon une variante du procédé, l'extrait végétal, éventuellement concentré, purifié, aromatisé et/ou coloré, est mélangé à la pâte ou à la bouillie avant le procédé papetier ou l'extrusion (14) formant un article (2b) par exemple une trame fibreuse.

Selon une autre variante du procédé, le façonnage d'un article selon l'invention, par exemple un bâtonnet ou un granulé est réalisé par un procédé d'extrusion. L'imprégnation par l'extrait végétal amélioré (3b) est alors réalisée simultanément ou postérieurement à la mise en forme des articles par l'extrudeur (14).

Selon un autre mode de réalisation de la présente invention relatif à la figure 2, le procédé selon l'invention comprend une étape d'extraction (10) et/ou pressage (9) suivie d'une étape de séparation (11) des produits insolubles ou résidus fibreux (R1) d'un végétal (V1) ou partie d'un végétal des substances solubles ou extrait végétal (E1).

Préalablement aux étapes d'extraction (10) et/ou pressage (9), une étape optionnelle de broyage/découpage (8) peut être mise en oeuvre. Elle a pour but de déchiqueter le végétal ou partie de végétal et donc de rompre les parois des cellules végétales.

L'extraction et/ou le pressage peut être réalisé(e) sur au moins une partie de végétal, frais, congelé ou sec, choisi parmi les racines, l'écorce, les graines, la tige, les feuilles, les fleurs et les fruits.

L'extraction (10) peut être mise en oeuvre par pressage (9) et/ou par extraction (10) de végétaux, en particulier de plantes, avec au moins un solvant (5) à température ambiante et à pression atmosphérique, ledit solvant pouvant être choisi parmi l'eau, l'éthanol, l'hexane, l'acétone et les hydrofluorocarbones. D'autres procédés d'extraction peuvent être utilisés mettant en jeu au moins un solvant, tel que par exemple le R134a ou alors le dioxyde de carbone et ce à différentes températures, à différentes pressions et différents états (liquides ou gazeux). On peut, par exemple, utiliser un solvant tel que par exemple choisi parmi les solvants mentionnés ci-dessus ou, le cas échéant le dioxyde de carbone :
- à l'état liquide (solvants volatils et non volatils à température ambiante),
- à l'état subcritique (l'eau à une température supérieure à 100°C et une pression supérieure à 1 bar), ou
- à l'état supercritique (le CO₂ à une température supérieure à 31 °C et une pression supérieure à 73 bars).

Le rapport pondéral solvant/végétal est en général de 1 à 10.

Ladite étape de séparation (11) peut être mise en oeuvre par séparation de la phase liquide de la phase solide résultantes par filtration, avec ou sans pression, par centrifugation ou par toutes autres méthodes couramment utilisées au laboratoire.

Selon un mode de réalisation, l'extraction solide-liquide est effectuée en utilisant un solvant dans lequel est mis en contact le végétal ou au moins une partie du végétal, éventuellement préalablement découpé(e) et/ou broyé(e), puis en séparant la phase liquide obtenue de la phase solide constituée par le végétal par filtration avec ou sans pression ou par centrifugation lors d'une étape de séparation.

Selon un autre mode de réalisation, l'extraction solide-liquide est effectuée en hydrodistillant le végétal ou au moins une partie du végétal, éventuellement découpé(e) et/ou broyé(e), avec l'eau comme solvant puis à séparer les phases organique et aqueuse résultantes.

Ces procédés d'extraction peuvent être couplés à une extraction mettant en oeuvre des micro-ondes tel que par exemple le procédé « Vacuum Microwave Hydrodistillation » abrégé VMHD, le procédé « Microwave Assisted Process » abrégé MAP et le procédé d'extraction par Solvant avec Micro-ondes abrégé ESAM, ou par l'utilisation des ultrasons tel que la sonication.

Ces procédés d'extraction peuvent être continus ou discontinus.

Les procédés et le cas échéant le ou les solvants peuvent être choisis pour satisfaire aux exigences de la réglementation des produits « bio » et être respectueux de l'environnement.

Une fois les substances solubles séparées des résidus fibreux, le procédé selon l'invention peut éventuellement comprendre une étape de concentration (15) des substances solubles ou extrait végétal en une liqueur, et/ou une étape d'isolement d'au moins une substance soluble de l'extrait ou de ladite liqueur, voire une étape de purification (16) ou d'élimination d'éventuelles impuretés présentes de manière à obtenir un extrait purifié, une liqueur purifiée ou au moins une substance soluble, isolée, purifiée. Les étapes de concentration, d'isolement et de purification se font selon les techniques habituellement utilisées en laboratoire.

Le procédé selon l'invention comprend, en outre, une étape de déstructuration (19) des produits insolubles ou résidus fibreux solides (R1) par un procédé qui peut être mécanique, chimique et/ou biologique, avantageusement un procédé mécanique couplé à un procédé biologique.

Le résidu fibreux peut être traité par un procédé mécanique d'agitation violente ou d'attrition et de délitation mécanique simple, lors d'une étape de trituration pouvant être assistée, par exemple, par des ultrasons. Le résidu fibreux peut ainsi être transformé en une bouillie, c'est-à-dire un appareil liquide inhomogène, avantageusement aqueux, contenant des fibres entières ou partiellement détruites ou en une pâte, c'est-à-dire un appareil liquide homogène, avantageusement aqueux, la consistance de l'appareil dépendant de l'intensité et la durée de cette étape de déstructuration.

Selon un mode de réalisation, l'étape de déstructuration peut avoir lieu par cisaillement mécanique et frottement du résidu fibreux entre un rotor et un stator, éventuellement accompagnés d'ultrasons.

Le résidu fibreux peut être traité biologiquement. Le résidu fibreux peut être (i) ensemencé par des levures, avantageusement des levures choisies dans le genre saccharomyces ou peut être (ii) mélangé à une quantité d'eau suffisante pour former une pâte liquide puis est ensemencé par des enzymes avantageusement des enzymes, choisies parmi les enzymes à activités pectinases, cellulases, amylases et leurs mélanges. On parle alors d'étape d'enzymage (12).

Cette étape biologique a pour but de réduire partiellement la taille des fibres notamment de cellulose, lignine et pectine.

Au moins un additif (7) peut être ajouté à l'extrait végétal (E1), éventuellement concentré, voire purifié. Ledit additif peut être choisi parmi (i) les arômes, comme par exemple le menthol, la réglisse, les extraits de fruits en général, (ii) les parfums et (iii) les colorants, comme par exemple le caramel, le rouge de betterave, les anthocyanes et le Cu-chlorophylline et/ou au moins un additif (7) d'origine naturelle peut être ajouté à la bouillie ou la pâte de résidu fibreux, ledit additif étant alors éventuellement choisi parmi (i) les liants tels que par exemple, la gomme arabique et la gomme guar, (ii) les hydrates de carbone d'origine végétale, éventuellement choisis parmi le glucose et les sucres invertis, (iii) les produits de source animale, éventuellement choisis parmi la chitine désacétylée et les gommes de plantes marines, telles que les alginates, les carraghénanes et l'agar-agar, (iv) les fibres de renforcement, comme par exemple des fibres de paille de céréales, de bagasse, de coton, de pin ou d'eucalyptus, (v) les arômes comme par exemple le menthol, la réglisse, les extraits de fruits en général, (vi) les colorants, avantageusement un colorant naturel hydrosoluble choisi par exemple parmi le caramel, le rouge de betterave, les anthocyanes et le Cu-chlorophylline , (vii) les parfums et (viii) les charges diverses.

Le procédé comprend ensuite une étape d'incorporation également appelée étape d'imprégnation (18) de (i) ladite liqueur de substances solubles éventuellement purifiées ou (ii) d'au moins ladite substance soluble isolée, éventuellement concentrée ou (iii) d'au moins une liqueur d'origine végétale ou (iv) d'un extrait végétal, à la bouillie ou la pâte, obtenues à l'issu de l'étape de déstructuration.

Le procédé selon l'invention comprend, ensuite, une étape de fabrication d'une trame fibreuse ou procédé papetier (13) conduisant à la formation d'un article fibreux imprégné, en particulier une feuille imprégnée.

Selon encore un mode de réalisation de la présente invention relatif à la figure 3, le procédé selon l'invention comprend une étape d'extraction des substances solubles ou extrait végétal (E1) d'un végétal (V1) par hydrodistillation avec l'eau comme solvant suivie d'une étape de décantation (24) des phases aqueuse et organique obtenues à l'issue de d'hydrodistillation, la phase organique contenant les huiles essentielles (26) dudit végétale (V1) et la phase aqueuse contenant l'eau florale (25) dudit végétale (V1).

On obtient ainsi la séparation des produits insolubles ou résidus fibreux (R1) d'un végétal (V1) ou partie d'un végétal des substances solubles à savoir l'eau florale (25) et les huiles essentielles (26).

Préalablement à l'étape d'hydrodistillation (10), une étape optionnelle de broyage/découpage (8) peut être mise en oeuvre. Elle a pour but de déchiqueter le végétal ou partie de végétal et donc de rompre les parois des cellules végétales.

L'hydrodistillation peut être réalisé(e) sur au moins une partie de végétal, frais, congelé ou sec, choisi parmi les racines, l'écorce, les graines, la tige, les feuilles, les fleurs et les fruits.

Le procédé selon l'invention comprend, en outre, une étape de déstructuration des produits insolubles ou résidus fibreux solides (R1) par un procédé qui peut être mécanique, chimique et/ou biologique, avantageusement un procédé mécanique couplé à un procédé biologique.

Le résidu fibreux peut être traité par un procédé mécanique d'agitation violente ou d'attrition et de délitation mécanique simple, lors d'une étape de trituration (19) pouvant être assistée, par exemple, par des ultrasons. Le résidu fibreux peut ainsi être transformé en une bouillie, c'est-à-dire un appareil liquide inhomogène, avantageusement aqueux, contenant des fibres entières ou partiellement détruites ou en une pâte, c'est-à-dire un appareil liquide homogène, avantageusement aqueux, la consistance de l'appareil dépendant de l'intensité et la durée de cette étape de déstructuration.

Selon un mode de réalisation, l'étape de déstructuration peut avoir lieu par cisaillement mécanique et frottement du résidu fibreux entre un rotor et un stator, éventuellement accompagnés d'ultrasons.

Le résidu fibreux peut être traité biologiquement. Le résidu fibreux peut être (i) ensemencé par des levures, avantageusement des levures choisies dans le genre saccharomyces ou peut être (ii) mélangé à une quantité d'eau suffisante pour former une pâte liquide puis est ensemencé par des enzymes lors d'une étape d'enzymage (12), avantageusement des enzymes choisies parmi les enzymes à activités pectinases, cellulases, amylases et leurs mélanges.

Cette étape biologique a pour but de réduire partiellement la taille des fibres notamment de cellulose, lignine et pectine. Par exemple, une certaine liquéfaction du résidu fibreux et l'élimination de ballast peut être obtenue par l'ajout d'au moins une enzyme choisie par exemple parmi les enzymes définies ci-dessus.

A l'issu de l'étape de trituration (19) ou de l'étape de trituration suivie de l'étape d'enzymage (12), on obtient une pâte définissant un appareil homogène ou une bouillie définissant un appareil inhomogène dû à une trituration grossière et incomplète.

La bouillie ou la pâte est alors imprégnée lors d'une étape d'imprégnation p(18) par l'eau florale (25). Elle peut, en outre, être éventuellement enrichie par au moins un additif (7) choisi parmi (i) les fibres de renforcement, comme par exemple des fibres de paille de céréales, de coton, ou d'eucalyptus, (ii) les humectants, comme par exemple le glucose et les sucres invertis, (iii) les arômes comme par exemple le menthol, la réglisse, les extraits de fruits en général, (iv) les colorants et (v) les charges diverses.

Puis la bouillie ou la pâte de résidu fibreux peut ensuite être transformée par des techniques courantes en papeterie selon un procédé papetier (13), de façon continue ou discontinue, en un article (2b) par exemple une trame fibreuse appelée également trame papetière.

Notamment par un procédé simple, la pâte ou la bouillie est étalée sur une bande métallique sans fin et séchée à l'air formant ainsi une feuille de papier. Mais de nombreux autres procédés de fabrication de feuilles de papier sont connus, par exemple les procédés par stratification ou par pressage.

Le procédé comprend enfin une étape d'imprégnation (18) de la trame fibreuse obtenue à l'issue du procédé papetier (13) par les huiles essentielles (26).

Lors de l'étape d'imprégnation (18) du résidu fibreux de la trame fibreuse, ce dernière peut être, en outre, imprégnée avec (i) au moins un colorant, avantageusement un colorant naturel hydrosoluble choisi parmi le caramel, le rouge de betterave, les anthocyanes et le Cu-chlorophylline et/ou avec (ii) au moins une fibre alimentaire hydrosoluble d'origine végétale ou animale choisie parmi les carraghénanes, les alginates, les pectines, les amidons et les xanthanes, les caséines et les gélatines, avantageusement une fibre alimentaire hydrosoluble d'origine végétale.

On obtient à l'issue de ce procédé un article fibreux imprégné, en particulier une feuille imprégnée.

Par une variante des procédés par voie humide avec extraction-réincorporation des substances d'intérêts, l'invention s'applique aussi à des matières végétales reconstituées qui seraient obtenues par voie sèche en mettant directement en contact des matières végétales pures ou en mélange sous forme pratiquement sèche avec ou sans agent liant et/ou autres substances ajoutées, puis mélangées et soumises à un taux élevé d'agitation de découpage et de dégradation en présence d'une teneur en eau relativement faible (taux d'humidité inférieur à 30% environ). La matière ainsi préparée étant traitée ultérieurement dans un appareil de mise en feuilles (pressage ou laminage) et de découpage utilisant des techniques par voie sèche de manière à obtenir des matières végétales reconstituées.

Les articles selon l'invention, susceptibles d'être obtenus selon l'un des modes de réalisation du procédé présenté ci-dessus, peuvent être, par exemple, des articles décoratifs tels que des articles en forme de feuilles végétales reconstituées, de fleurs,... ou alors peuvent être des articles tels que des feuilles de papier de différentes épaisseurs, des filaments, des granulés, des chips ou des tubes. Ces articles peuvent éventuellement être broyés permettant de générer des particules de poudre de plantes déstructurées et imprégnées.

Avantageusement, la poudre est destinée à être incorporée dans des compositions pharmaceutiques, des compositions cosmétiques, des compositions alimentaires, des compositions diététiques.

Le procédé peut être utilisé avec des plantes en mélange autorisant la recherche de synergie originale comme cela a déjà été observé en médecine chinoise et médecine ayurvédique.

Les articles susceptibles d'être obtenus par le procédé selon l'invention ont l'avantage :
- de pouvoir être utilisés directement pour fabriquer par exemple des tisanes ou des infusions pouvant être à vertus médicinales, cosmétiques, diététiques, nutraceutiques,
- ou alors finement broyés, de pouvoir être incorporés (i) dans des formes galéniques, telles que par exemple des gélules, ou encore (ii) dans de nombreuses compositions pour des applications topiques ou orales (par exemples, dans des sirops, dans des gommes à mâcher, dans des gels, des lotions, des crèmes, des émulsions), lesdites compositions pouvant être destinées à être utilisées à des fins alimentaires, diététiques, médicinales, nutraceutiques ou cosmétiques

Les articles objets de l'invention ont en outre l'avantage par rapport aux produits traditionnellement utilisés d'être de grande qualité car les substances solubles qui pourraient être partiellement détruites ou totalement modifiées par les procédés de trituration et de fabrication papetière sont temporairement séparées de la fraction fibreuse soumise aux traitements. Notamment par cette opération, les propriétés sensorielles des plantes sont remarquablement conservées.

Ils peuvent être dosables en principes actifs lors de l'incorporation de la liqueur ou de l'extrait végétal dans la trame fibreuse papetière et dépourvu de substances gênantes (par exemple les pesticides, substances toxiques, et autres allergisants) éventuellement éliminées au cours du procédé.

Les matières premières végétales utilisées pour fabriquer les articles selon l'invention peuvent être constituées de végétaux entiers ou de parties de végétaux notamment de tiges, écorces, graines et de déchets de végétaux (poussières, côtes et débris). Les matières premières peuvent être à l'état sec, humide, frais, fermenté ou torréfié.

Les articles selon l'invention ont une structure homogène (pas de nervures) et peuvent présenter une surface plane et constante ayant un bon rendu visuel.

Les principes actifs sont facilement extractibles des articles selon l'invention par l'organisme humain en cas d'ingestion ou de mise en contact sur la peau ou les cheveux, car les éléments pouvant s'opposer à une extraction aisée (fibres, membranes, tannins,...) ont été éliminés ou modifiés. Ce même résultat est observé lors de l'utilisation des articles selon l'invention en tisanes. Par conséquent, les produits objets de l'invention lorsqu'ils sont réduits en poudres, se distinguent fondamentalement des poudres disponibles sur le marché fabriquées par le seul broyage de plantes ou parties de plantes (cryobroyage, micronisation,..) sans traitement déstructurant du ballast entourant les substances actives d'intérêt.

La teneur contrôlée en eau du produit fini et le procédé selon l'invention garantissent une meilleure qualité bactériologique que les plantes, tisanes et poudres utilisées traditionnellement telles quelles sans décontamination physique ou chimique.

Le procédé selon l'invention permet de fabriquer des feuilles de papier d'épaisseur variable qui peuvent être découpées par des emportes pièces de formes variées et peuvent aussi prendre une forme tridimensionnelle par moulage ou par extrusion, permettant éventuellement de former des articles à formes décoratives, avec une densité et une perméabilité précisément définies et adaptées à leur utilisation finale.

Le procédé selon l'invention peut être réalisé sans ajout d'aucune substance autre que des végétaux ou parties de végétaux et de l'eau (procédé « bio » et 100% naturel) mais néanmoins, si cela est justifié le procédé autorise l'addition homogène dans le produit fini de substances par exemple aromatiques, colorantes, d'autres actifs (antioxydants, vitamines, ...), des liants et si nécessaire des fibres de renfort (paille de céréales, fibres de bagasse, lin ou coton...).

D'autres avantages pourront encore apparaître à l'homme du métier à la lecture des exemples ci-dessous, illustrés par les figures annexées, donnés à titre illustratif.

### EXEMPLES

### Exemple 1 : (voir figure 2)

Le procédé consiste à broyer grossièrement par cisaillement des sommités fleuries (fleurs, tiges et feuilles) de menthe fraîche (Mentha x piperita) et à extraire à l'eau les principes actifs (rapport plantes broyées/eau : 1/5, à une température maintenue à 60-70°C pendant 15 minutes avec agitation) puis à séparer par pressage mécanique l'extrait soluble des parties insolubles. L'opération est renouvelée 2 fois.

Le premier extrait est concentré à moitié par évaporation et la fraction fibreuse est triturée en présence d'eau (dispersion/homogénéisation rotor/stator par un Ultra-Turrax IKA) jusqu'à former une pâte.

Une solution liquide de menthol à 10% est préparée et maintenue à 40°C.

Les produits pour 1% et le concentré en totalité sont ensuite mélangés aux insolubles de menthe déstructurés seule ou en mélange avec des feuilles de thé (Camelia sinensis) finement pulvérisées et/ou des feuilles et tiges de Stevia rebaudiana et à former par coulage ou extrusion dudit mélange une feuille reconstituée. La feuille peut être ensuite séchée et découpée. La feuille découpée est utilisée telle quelle en infusion traditionnelle ou en infusette après broyage. La teneur du produit en menthol est très élevée. Dans une variante du procédé des huiles essentielles de menthe se substituent à la solution de menthol.

### Exemple 2 : (voir figure 3)

Le procédé consiste à hydrodistiller à pression atmosphérique 100g de sommités fleuries de Romarin (Rosmarinus officinalis) séchées (mélange de feuilles coriaces, tiges fibreuses, brindilles et fleurs) par 2l d'eau à l'état de vapeur. Puis après condensation de la vapeur d'eau et refroidissement, le procédé consiste à séparer la phase supérieure, soit environ 0,5 ml d'huile essentielle, de la phase inférieure (l'eau blanche pour environ 1,5l).

L'huile essentielle est un liquide très fluide incolore à jaune pâle. Elle est connue pour contenir du bornéol du cinéol, du camphène du pinène et de l'acide chlorogénique. L'acide rosmarinique qui est un antioxydant puissant se partage entre la phase aqueuse, l'huile essentielle et le résidu solide. L'eau florale ou eau blanche est concentrée 5 fois sous vide à une température ne dépassant pas 60°C puis elle est ajoutée au résidu insoluble de la distillation du romarin.

L'ensemble est agité violemment (Ultra turax IKA) jusqu'à former une pâte laquelle est ensemencée par un cocktail enzymatique (Peclyve CP et EP de Lyven en mélange 50/50) à 1% en poids pendant 3 heures à 45°C. La bouillie est ensuite coulée dans un moule et pressée. Après séchage à l'air chaud, le film formé est imprégné par pulvérisation avec l'huile essentielle en solution alcoolique.

## Revendications

1. Procédé de fabrication d'articles imprégnés d'au moins une substance végétale à partir d'au moins un végétal **caractérisé en ce qu'**il comprend les étapes suivantes :
a) Extraction et/ou pressage d'au moins un végétal (V1) ou d'au moins une partie dudit végétal produisant un extrait végétal liquide (E1) et un résidu fibreux solide (R1), puis
b) Séparation dudit extrait végétal (E1) dudit résidu fibreux (R1), et
c) Déstructuration dudit résidu fibreux (R1),
d) Fabrication d'une trame fibreuse ou d'un article façonné à partir du résidu fibreux (R1) obtenu à l'étape c),
e) Imprégnation dudit résidu fibreux (R1), avec (i) au moins ledit extrait végétal (E1), éventuellement concentré, purifié, aromatisé et/ou parfumé, ou avec (ii) au moins une substance végétale hydrosoluble ou liposoluble isolée dudit extrait végétal (E1), ou avec (iii) au moins une composition comprenant au moins une substance hydrosoluble ou liposoluble dudit extrait végétal (E1) éventuellement concentrée, purifiée, aromatisée et/ou parfumée.

2. Procédé selon la revendication 1, dans lequel les étapes c), d) et e) peuvent avoir lieux dans l'ordre suivant :
l'étape c) précède l'étape d) qui elle-même précède l'étape e), ou
l'étape c) précède l'étape e) qui précède l'étape d).

3. Procédé selon l'une quelconque des revendications précédentes, dans lequel :
le végétal est choisi parmi les plantes alimentaires, médicinales, les plantes aromatiques et les plantes à parfum ; ou dans lequel
le végétal est choisi parmi les végétaux contenant au moins une substance choisie parmi les antioxydants, les édulcorants, les parfums, les arômes, les caroténoïdes, les xantophylles, les colorants, les flavonoïdes, les tanins, les polyphénols, les peptides, les vitamines, les protéines et les principes actifs pharmaceutiques, et/ou dans lequel
le végétal est choisi parmi le romarin, la sauge, le thym, la menthe, l'origan, le curcuma, le basilic, les clous de girofle, le stevia rebaudiana, l'ail, le thé, le café, le saule, le ginseng, le gingko, la vigne rouge, le thé vert et l'artémisia.

4. Procédé selon la revendication précédente, dans lequel le principe actif pharmaceutique est choisi parmi la salicine de l'écorce de saule, les gingkolides des feuilles de gingko biloba, l'hyperforine des somités fleuries du millepertuis, l'artemisinine des feuilles et tiges de artemisia annua, la curcumine des racines de curcuma longa, la genistéïne et la daidzéine des graines de soja, les gingsenoïdes des racines de ginseng, les anthocyanosides et les tanins des feuilles de vigne rouge et les steviosides des feuilles de stevia rebaudiana.

5. Procédé selon l'une quelconque des revendications précédentes, dans lequel l'étape a) d'extraction et/ou de pressage est réalisée sur au moins une partie de végétal, frais, congelé ou sec, choisi parmi les racines, l'écorce, les graines, la tige, les feuilles, les fleurs et les fruits.

6. Procédé selon l'une quelconque des revendications précédentes, dans lequel l'extraction est une extraction choisie parmi l'extraction au CO₂ supercritique, l'extraction solide-liquide en utilisant un solvant et l'extraction subcritique, éventuellement couplé à ou se présenter sous la forme d'une extraction mettant en oeuvre des ultra-sons ou des micro-ondes, éventuellement
dans lequel le solvant est choisi parmi l'eau, l'éthanol et l'hexane, et/ou dans lequel le rapport pondéral solvant/végétal est de 1 à 10.

7. Procédé selon la revendication 6, dans lequel l'extraction solide-liquide est effectuée en utilisant un solvant dans lequel est mis en contact le végétal ou au moins une partie du végétal, éventuellement préalablement découpé(e) et/ou broyé(e), puis en séparant la phase liquide obtenue de la phase solide constituée par le végétal par filtration avec ou sans pression ou par centrifugation.

8. Procédé selon la revendication 6, dans lequel l'extraction solide liquide est effectuée en hydrodistillant le végétal ou au moins une partie du végétal, éventuellement découpé(e) et/ou broyé(e), avec l'eau comme solvant puis à séparer les phases organique et aqueuse résultantes.

9. Procédé selon l'une quelconque des revendications précédentes, **caractérisé en ce que** l'étape de déstructuration s'opère selon un procédé mécanique, chimique et/ou biologique.

10. Procédé selon l'une quelconque des revendications précédentes, dans lequel l'étape de déstructuration a lieu par cisaillement mécanique et frottement du résidu fibreux entre un rotor et un stator éventuellement accompagnés d'ultrasons.

11. Procédé selon l'une quelconque des revendications précédentes, dans lequel on rajoute au résidu fibreux (R1) des fibres alimentaires avantageusement des fibres choisies parmi les fibres de paille de céréales, de bagasse, de coton, de pin et d'eucalyptus.

12. Procédé selon l'une quelconque des revendications précédentes, dans lequel, le résidu fibreux (R1) est (i) ensemencé par des levures, avantageusement des levures choisies dans le genre saccharomyces ou est (ii) ensemencé par des enzymes avantageusement des enzymes choisies parmi les enzymes à activités pectinases, cellulases, amylases et leurs mélanges.

13. Procédé selon l'une quelconque des revendications précédentes, dans lequel l'étape de fabrication d'un article façonné se fait par extrusion.

14. Procédé selon l'une quelconque des revendications précédentes, dans lequel lors de l'étape de façonnage les articles façonnés se présentent sous la forme de feuilles, de bâtonnets, de granulés, de fibres, pleines ou creuses, ou de chips.

15. Procédé selon l'une quelconque des revendications précédentes, dans lequel lors de l'étape d'imprégnation e) du résidu fibreux, ce dernière est, en outre, imprégnée avec (i) au moins un colorant, avantageusement un colorant naturel hydrosoluble choisi parmi le caramel, le rouge de betterave, les anthocyanes et le Cu-chlorophylline et/ou avec (ii) au moins une fibre alimentaire hydrosoluble d'origine végétale ou animale choisie parmi les carraghénanes, les alginates, les pectines, les amidons et les xanthanes, les caséines et les gélatines, avantageusement une fibre alimentaire hydrosoluble d'origine végétale.

16. Article d'origine végétale susceptible d'être obtenu par le procédé selon l'une des revendications précédentes, comprenant une structure solide compacte, homogène, de fibres végétales, ladite structure étant imprégnée par (i) au moins un extrait végétal, éventuellement concentré, purifié, aromatisé, coloré et/ou parfumé, ou (ii) au moins une substance végétale hydrosoluble ou liposoluble isolée de l'extrait végétal, ou (iii) au moins une composition comprenant au moins une substance hydrosoluble ou liposoluble de l'extrait végétal éventuellement concentrée, purifiée, aromatisée, coloré et/ou parfumée, dans lequel ladite structure et ledit extrait végétal ont la même origine végétale.

17. Article selon la revendication 16, dans lequel l'extrait d'origine végétal est choisi parmi les extraits de racines, d'écorce, de graines, de tige, de feuilles, de fleurs , de fruits ou leurs mélanges et/ou dans lequel :
le végétal est choisi parmi les plantes alimentaires, médicinales, les plantes aromatiques et les plantes à parfum, et/ou dans lequel
le végétal est choisi parmi les végétaux contenant au moins une substance choisie parmi les antioxydants, les édulcorants, les parfums, les arômes, les caroténoïdes, les xantophylles, les colorants, les flavonoïdes, les tanins, les polyphénols, les peptides, les vitamines, les protéines et les principes actifs pharmaceutiques, et/ou dans lequel
le végétal est choisi parmi le romarin, la sauge, le thym, la menthe, l'origan, le curcuma, le basilic, les clous de girofle, le stevia rebaudiana, l'ail, le thé, le café, le saule, le ginseng, le gingko, la vigne rouge, le thé vert et l' artémisia.

18. Article selon l'une quelconque des revendications 16 à 17, imprégné en outre par au moins un colorant, avantageusement un colorant naturel hydrosoluble choisi parmi le caramel, le rouge de betterave, les anthocyanes et le Cu-chlorophylline et/ou par (ii) au moins une fibre alimentaire hydrosoluble d'origine végétale ou animale choisie parmi les carraghénanes, les alginates, les pectines, les amidons et les xanthanes, les caséines et les gélatines, avantageusement une fibre alimentaire hydrosoluble d'origine végétale.

19. Utilisation des articles selon l'une quelconques des revendications 16 à 18, en agroalimentaire, phytothérapie, cosmétique, pharmacie, herboristerie, nutraceutique et tisanerie.

20. Utilisation selon la revendication 19 pour fabriquer une poudre destinée à être incorporée dans des compositions pharmaceutiques, des compositions cosmétiques, des compositions alimentaires, des compositions diététiques.

## Patentansprüche

1. Verfahren zur Herstellung von Gegenständen, die mit mindestens einer pflanzlichen Substanz durchtränkt sind, ausgehend von mindestens einer Pflanze, **dadurch gekennzeichnet, dass** es die folgenden Schritte umfasst:
a) Extrahieren und/oder Pressen mindestens einer Pflanze (V1) oder mindestens eines Teils der Pflanze, wobei ein flüssiger Pflanzenextrakt (E1) und ein feststofflicher faserartiger Rückstand (R1) erhalten wird, und im Anschluss daran
b) Abtrennen des Pflanzenextrakts (E1) vom faserartigen Rückstand (R1), und
c) Aufschließen des faserartigen Rückstands (R1),
d) Herstellen einer faserartigen Stützstruktur oder eines geformten Gegenstands aus dem faserartigen Rückstand (R1), der in Schritt c) erhalten wurde,
e) Durchtränken des faserartigen Rückstands (R1) mit (i) mindestens dem Pflanzenextrakt (E1), der möglicherweise aufkonzentriert, aufgereinigt, aromatisiert und/oder mit Duftstoffen versetzt wurde, oder mit (ii) mindestens einer wasserlöslichen oder fettlöslichen pflanzlichen Substanz, die aus dem Pflanzenextrakt (E1) isoliert wurde, oder mit (iii) mindestens einer Zusammensetzung, die mindestens eine wasserlösliche oder fettlösliche Substanz des Pflanzenextrakts (E1) umfasst, welche möglicherweise aufkonzentriert, aufgereinigt, aromatisiert und/oder mit Duftstoffen versetzt wurde.

2. Verfahren nach Anspruch 1, wobei die Schritte c), d) und e) in der folgenden Reihenfolge erfolgen können:
Schritt c) geht dem Schritt d) voran, welcher wiederum dem Schritt e) vorangeht, oder
Schritt c) geht dem Schritt e) voran, welcher wiederum dem Schritt d) vorangeht.

3. Verfahren nach einem beliebigen der vorhergehenden Ansprüche, wobei:
die Pflanze aus den Nahrungsmittelpflanzen, den Heilpflanzen, den Küchenkräutern und Gewürzpflanzen sowie den duftenden Pflanzen ausgewählt ist; oder wobei
die Pflanze aus den Pflanzen ausgewählt ist, die mindestens eine Substanz enthalten, welche aus den Antioxidantien, den Süßungsmitteln, den Duftstoffen, den Aromastoffen, den Carotinoiden, den Xanthophyllen, den Farbstoffen, den Flavonoiden, den Tanninen, den Polyphenolen, den Peptiden, den Vitaminen, den Proteinen und den pharmazeutischen Wirkstoffen ausgewählt ist, und/oder wobei
die Pflanze aus Rosmarin, Salbei, Thymian, Minze, Oregano, Kurkuma, Basilikum, Gewürznelken, Stevia rebaudiana, Knoblauch, Tee, Kaffee, Weide, Ginseng, Ginkgo, Weinreben, grünem Tee und Artemisia ausgewählt ist.

4. Verfahren nach dem vorhergehenden Anspruch, wobei der pharmazeutische Wirkstoff aus dem Salicin der Weidenrinde, den Ginkgoliden der Blätter von Ginkgo biloba, dem Hyperforin der Blütenstände des Johanniskrauts, dem Artemisin der Blätter und Stiele von Artemisia annua, dem Kurkumin der Wurzeln von Curcuma longa, dem Genistein und dem Daidzein der Sojasamen, den Ginsenoiden der Wurzeln des Ginseng, den Anthocyanosiden und den Tanninen der Blätter der Weinrebe sowie den Steviosiden der Blätter von Stevia rebaudiana ausgewählt ist.

5. Verfahren nach einem beliebigen der vorhergehenden Ansprüche, wobei der Schritt a) der Extraktion und/oder der Pressung an mindestens einem Teil der Pflanze vorgenommen wird, wobei dieser frisch, tiefgefroren oder getrocknet ist und aus den Wurzeln, der Rinde, den Samen, dem Stiel, den Blättern, den Blüten und den Früchten ausgewählt ist.

6. Verfahren nach einem beliebigen der vorhergehenden Ansprüche, wobei es sich bei der Extraktion um eine Extraktion handelt, die aus der Extraktion mit überkritischem CO₂, der fest-flüssig-Extraktion unter Verwendung eines Lösungsmittels und der unterkritischen Extraktion ausgewählt ist, wobei diese möglicherweise in Form einer Extraktion durchgeführt wird, bei welcher Ultraschall oder Mikrowellen zum Einsatz kommen, oder möglicherweise an eine solche gekoppelt ist.
wobei das Lösungsmittel aus Wasser, Ethanol und Hexan ausgewählt ist und/oder wobei das Gewichtsverhältnis Lösungsmittel/Pflanze im Bereich von 1 bis 10 liegt.

7. Verfahren nach Anspruch 6, wobei die fest-flüssig-Extraktion unter Verwendung eines Lösungsmittels durchgeführt wird, in welchem eine Berührung mit der Pflanze oder mindestens einem Teil der Pflanze erfolgt, nachdem diese(r) möglicherweise zuvor zerschnitten und/oder zerkleinert wurde, woraufhin die erhaltene flüssige Phase durch Filtration mit oder ohne Druckeinwirkung oder durch Zentrifugation von der festen Phase getrennt wird, welche aus der Pflanze besteht.

8. Verfahren nach Anspruch 6, wobei die fest-flüssig-Extraktion durchgeführt wird, indem die Pflanze oder mindestens ein Teil der Pflanze, welche(r) möglicherweise zerschnitten und/oder zerkleinert wurde, mit Wasser als Lösungsmittel einer Wasserdampfdestillation unterzogen wird, um anschließend die erhaltenen organischen und wässrigen Phasen zu trennen.

9. Verfahren nach einem beliebigen der vorhergehenden Ansprüche, **dadurch gekennzeichnet, dass** der Aufschlussschritt gemäß einer mechanischen, chemischen und/oder biologischen Vorgehensweise erfolgt.

10. Verfahren nach einem beliebigen der vorhergehenden Ansprüche, wobei der Aufschlussschritt durch mechanische Scherwirkung oder Reiben des faserartigen Rückstands zwischen einem Rotor und einem Stator erfolgt, wobei möglicherweise zusätzlich Ultraschall einwirkt.

11. Verfahren nach einem beliebigen der vorhergehenden Ansprüche, wobei dem faserartigen Rückstand (R1) Nahrungsfasern zugesetzt werden, vorteilhafterweise Fasern, die aus den Fasern von Getreidestroh, von Bagasse, von Baumwolle, von Kiefern und von Eukalyptus ausgewählt sind.

12. Verfahren nach einem beliebigen der vorhergehenden Ansprüche, wobei der faserartige Rückstand (R1) (i) mit Hefen beimpft wird, vorteilhafterweise mit Hefen, die aus der Gattung Saccharomyces ausgewählt sind, oder (ii) mit Enzymen beimpft wird, vorteilhafterweise mit Enzymen, die aus den Enzymen mit Pectinase-, Cellulase-, Amylase-Aktivität und aus deren Mischungen ausgewählt sind.

13. Verfahren nach einem beliebigen der vorhergehenden Ansprüche, wobei der Schritt des Herstellens eines geformten Gegenstands mittels Extrusion erfolgt.

14. Verfahren nach einem beliebigen der vorhergehenden Ansprüche, wobei die geformten Gegenstände beim Formungsschritt als Blätter, Stäbchen, Granulatkörner, Voll- oder Hohlfasern oder als Schnitzel vorliegen.

15. Verfahren nach einem beliebigen der vorhergehenden Ansprüche, wobei beim Schritt e) des Durchtränkens des faserartigen Rückstands der letztere weiterhin mit (i) mindestens einem Farbstoff durchtränkt wird, vorteilhafterweise einem wasserlöslichen natürlichen Farbstoff, der aus Karamell, Betenrot, Anthocyanen und Cu-Chlorophyllin ausgewählt ist, und/oder mit (ii) mindestens einer wasserlöslichen Nahrungsfaser pflanzlichen oder tierischen Ursprungs, die aus den Carrageen, den Alginaten, den Pektinen, den Stärken sowie den Xanthanen, den Caseinen und den Gelatinen ausgewählt ist, vorteilhafterweise mit einer wasserlöslichen Nahrungsfaser pflanzlichen Ursprungs.

16. Gegenstand pflanzlichen Ursprungs, erhältlich durch das Verfahren nach einem der vorhergehenden Ansprüche, wobei er eine homogene, kompakte feststoffliche Struktur aus pflanzlichen Fasern umfasst, wobei die Struktur mit (i) mindestens einem Pflanzenextrakt durchtränkt ist, welcher möglicherweise aufkonzentriert, aufgereinigt, aromatisiert, gefärbt und/oder mit Duftstoffen versetzt wurde, oder mit (ii) mindestens einer wasserlöslichen oder fettlöslichen pflanzlichen Substanz, die aus dem Pflanzenextrakt isoliert wurde, oder mit (iii) mindestens einer Zusammensetzung, die mindestens eine wasserlösliche oder fettlösliche Substanz des Pflanzenextrakts umfasst, welche möglicherweise aufkonzentriert, aufgereinigt, aromatisiert, gefärbt und/oder mit Duftstoffen versetzt wurde, wobei die Struktur und der Pflanzenextrakt denselben pflanzlichen Ursprung haben.

17. Gegenstand nach Anspruch 16, wobei der Extrakt pflanzlichen Ursprungs aus den Extrakten von Wurzeln, von Rinde, von Samen, des Stiels, von Blättern, von Blüten, von Früchten oder deren Mischungen ausgewählt ist, und/oder wobei:
die Pflanze aus den Nahrungsmittelpflanzen, den Heilpflanzen, den Küchenkräutern und Gewürzpflanzen sowie den duftenden Pflanzen ausgewählt ist; und/oder wobei
die Pflanze aus den Pflanzen ausgewählt ist, die mindestens eine Substanz enthalten, welche aus den Antioxidantien, den Süßungsmitteln, den Duftstoffen, den Aromastoffen, den Carotinoiden, den Xanthophyllen, den Farbstoffen, den Flavonoiden, den Tanninen, den Polyphenolen, den Peptiden, den Vitaminen, den Proteinen und den pharmazeutischen Wirkstoffen ausgewählt ist, und/oder wobei
die Pflanze aus Rosmarin, Salbei, Thymian, Minze, Oregano, Kurkuma, Basilikum, Gewürznelken, Stevia rebaudiana, Knoblauch, Tee, Kaffee, Weide, Ginseng, Ginkgo, Weinreben, grünem Tee und Artemisia ausgewählt ist.

18. Gegenstand nach einem beliebigen der Ansprüche 16 bis 17, der weiterhin mit mindestens einem Farbstoff durchtränkt wird, vorteilhafterweise einem wasserlöslichen natürlichen Farbstoff, der aus Karamell, Betenrot, Anthocyanen und Cu-Chlorophyllin ausgewählt ist, und/oder mit (ii) mindestens einer wasserlöslichen Nahrungsfaser pflanzlichen oder tierischen Ursprungs, die aus den Carrageen, den Alginaten, den Pektinen, den Stärken sowie den Xanthanen, den Caseinen und den Gelatinen ausgewählt ist, vorteilhafterweise mit einer wasserlöslichen Nahrungsfaser pflanzlichen Ursprungs.

19. Verwendung der Gegenstände nach einem beliebigen der Ansprüche 16 bis 18, im Bereich der Ernährung, Phytotherapie, Kosmetik, Pharmazie, Pflanzenheilkunde, der funktionellen Lebensmittel und der Kräuterteeherstellung.

20. Verwendung nach Anspruch 19 zur Herstellung eines Pulvers, das dazu bestimmt ist, pharmazeutischen Zusammensetzungen, kosmetischen Zusammensetzungen, Nahrungsmittelzusammensetzungen, diätetischen Zusammensetzungen beigefügt zu werden.

## Claims

1. Method for manufacturing articles impregnated with at least one plant substance from at least one plant, **characterised in that** said method comprises the following steps:
a) Extraction and/or pressing at least one plant (V1) or at least one part of said plant so as to produce a liquid plant extract (E1) and a solid fibrous residue (R1), then
b) Separation of said plant extract (E1) from said fibrous residue (R1), and
c) Destructuring said fibrous residue (R1),
d) Producing a fibrous web or an article made from the fibrous residue (R1) obtained in step c),
e) Impregnating said fibrous residue (R1) with (i) at least said plant extract (E1) that is optionally concentrated, purified, flavoured and/or fragranced, or with (ii) at least one water-soluble or lipo-soluble plant substance isolated from said plant extract (E1), or with (iii) at least one composition comprising at least one optionally concentrated, purified, flavoured and/or fragranced water-soluble or lipo-soluble substance of said plant extract (E1).

2. Method according to claim 1, wherein the steps c), d) and e) may take place in the following order:
step c) precedes step d) that itself precedes step e), or
step c) precedes step e) that precedes step d).

3. Method according to one of the preceding claims, wherein:
the plant is chosen from among food plants, medicinal plants, aromatic plants and perfume plants; or wherein
the plant is chosen from among plants containing at least one substance chosen from among antioxidants, sweetening agents, perfumes, flavours, carotenoids, xanthophylls, colourants, flavonoids, tannins, polyphenols, peptides, vitamins, proteins, and pharmaceutical active ingredients, and/or wherein
the plant is chosen from among rosemary, sage, thyme, mint, oregano, turmeric, basil, cloves, *stevia rebaudiana,* garlic, tea, coffee, willow, ginseng, gingko, red vine, green tea and artemisia.

4. Method according to the preceding claim, wherein the pharmaceutical active ingredient is chosen from among salicin from willow bark, gingkolides from *gingko biloba* leaves, hyperforin from St. John's wort blossom, artemisinin from the leaves and stems of *artemisia annua*, curcumin from *curcuma longa* roots, genistein and daidzein from soya seeds, gingsenoides from ginseng roots, anthocyanosides and tannins from red vine leaves and steviosides from *stevia rebaudiana* leaves.

5. Method according to one of the preceding claims, wherein the extraction and/or pressing step a) is carried out on at least one fresh, frozen or dry plant part, chosen from among the roots, bark, seeds, stem, leaves, flowers and fruits.

6. Method according to one of the preceding claims, wherein the extraction is an extraction chosen from among extraction with supercritical CO₂, solid-liquid extraction using a solvent and subcritical extraction, optionally coupled with or assuming the form of an extraction involving ultrasound or microwaves, optionally
wherein the solvent is chosen among water, ethanol and hexane, and/or wherein the solvent/plant weight ratio is from 1 to 10.

7. Method according to claim 6, wherein the solid-liquid extraction is done by using a solvent that is brought into contact with the plant or at least part of the plant, optionally cut and/or ground beforehand, the liquid phase obtained from the solid phase that consists of the plant is then separated by filtration with or without pressure or by centrifugation.

8. Method according to claim 6, wherein the solid-liquid extraction is carried out by hydrodistillation of the plant or at least part of the plant, optionally cut and/or ground, with water as the solvent, and the resulting organic and aqueous phases are then separated.

9. Method according to one of the preceding claims, **characterised in that** the destructuring step is carried out by the use of a mechanical, chemical and/or biological method.

10. Method according to one of the preceding claims, wherein the destructuring step occurs by mechanical shearing and friction of the fibrous residue between a rotor and a stator, optionally accompanied by ultrasounds.

11. Method according to one of the preceding claims, wherein food fibres, advantageously fibres chosen from among cereal straw, bagasse, cotton, pine and eucalyptus fibres, are added to the fibrous residue (R1).

12. Method according to one of the preceding claims, wherein the fibrous residue (R1) is (i) seeded with yeasts, advantageously with yeasts chosen from the Saccharomyces genus or is (ii) seeded with enzymes, advantageously enzymes chosen from among enzymes with pectinase, cellulase, amylase activities and mixtures thereof.

13. Method according to one of the preceding claims, wherein the step for producing a produced article is carried out by extrusion.

14. Method according to one of the preceding claims, wherein the articles produced in the production step are in the form of sheets, rods, pellets, solid or hollow fibres, or chips.

15. Method according to one of the preceding claims, wherein during the impregnation step e) of the fibrous residue, the latter is also impregnated with (i) at least one colourant, advantageously at least one water-soluble natural colourant chosen from among caramel, beet red, anthocyanins and copper-chlorophyllin and/or with (ii) at least one water-soluble food fibre of plant or animal origin chosen from among carrageenans, alginates, pectins, starches and xanthans, caseins and gelatines, advantageously a water-soluble food fibre of plant origin.

16. Article of plant origin obtainable by the method of one of the preceding claims, comprising a compact, homogeneous solid structure of plant fibres, said structure being impregnated with (i) at least one optionally concentrated, purified, flavoured, coloured and/or fragranced plant extract, or (ii) at least one water-soluble or lipo-soluble plant substance isolated from a plant extract, or (iii) at least one composition comprising at least one water-soluble or lipo-soluble substance of an optionally concentrated, purified, flavoured, coloured and/or fragranced plant extract, wherein said structure and said plant extract have the same plant origin.

17. Article according to claim 16, wherein the extract of plant origin is chosen from among the extracts of roots, bark, seeds, stems, leaves, flowers, fruits or mixtures thereof and/or wherein:
the plant is chosen from among food plants, medicinal plants, aromatic plants and perfume plants, and/or wherein
the plant is chosen from among plants containing at least one substance chosen from among antioxidants, sweetening agents, perfumes, flavours, carotenoids, xanthophylls, colourants, flavonoids, tannins, polyphenols, peptides, vitamins, proteins, and pharmaceutical active ingredients, and/or wherein
the plant is chosen from among rosemary, sage, thyme, mint, oregano, turmeric, basil, cloves, *stevia rebaudiana,* garlic, tea, coffee, willow, ginseng, gingko, red vine, green tea and artemisia.

18. Article according to one of claims 16 to 17, impregnated with at least one colourant, advantageously at least one water-soluble natural colourant chosen from among caramel, beet red, anthocyanins and copper-chlorophyllin and/or with (ii) at least one water-soluble food fibre of plant or animal origin chosen from among carrageenans, alginates, pectins, starches and xanthans, caseins and gelatines, advantageously a water-soluble food fibre of plant origin.

19. Use of the articles according to one of claims 16 to 18 in the agri-food sector, phytotherapy, cosmetics, pharmacy, herbal uses, nutraceutical products and herbal teamaking.

20. Use according to claim 19 to produce a powder intended to be incorporated into pharmaceutical compositions, cosmetic compositions, food compositions, dietary compositions.
